# EUROPÄISCHE PATENTSCHRIFT

(11) **EP 2 725 989 B1**
(45) Veröffentlichungstag und Bekanntmachung des Hinweises auf die Patenterteilung: **05.09.2018**
(21) Anmeldenummer: 12730797.3
(22) Anmeldetag: 12.06.2012
(51) Int. Cl.: A61B 17/34

(54) **TROKARSYSTEM**
TROCAR SYSTEM
SYSTÈME DE TROCART

(30) Priorität: 30.06.2011 DE 102011107615
(43) Veröffentlichungstag der Anmeldung: 07.05.2014
(73) Patentinhaber: Riek, Siegfried, 78628 Rottweil (DE); Bachmann, Karl-Heinz, 78667 Villingendorf (DE); Gaiselmann, Thomas, 78667 Villingendorf (DE)
(72) Erfinder: Riek, Siegfried, 78628 Rottweil (DE); Bachmann, Karl-Heinz, 78667 Villingendorf (DE); Gaiselmann, Thomas, 78667 Villingendorf (DE)
(74) Vertreter: Westphal, Mussgnug & Partner Patentanwälte mbB
(86) Internationale Anmeldenummer: PCT/EP2012/002479
(87) Internationale Veröffentlichungsnummer: WO 2013/000540

(56) Entgegenhaltungen:
- WO-A1-2004/112596
- WO-A2-2006/050047
- US-A- 4 877 033
- US-A- 5 020 514
- US-A- 6 030 365
- US-A1- 2007 049 963
- US-A1- 2007 270 752
- US-A1- 2008 188 868
- US-A1- 2008 294 184

## Beschreibung

Die Erfindung betrifft ein Trokarsystem gemäß dem Oberbegriff des Patentanspruchs 1.

Trokarsysteme für die minimal-invasive Chirurgie bestehen in der Regel aus einem Trokardorn, um eine Öffnung in eine Körperhöhle (z.B. Bauchraum) zu schaffen, und einer Trokarhülse, die in dieser Öffnung verbleibt und einen Zugang zu dem Inneren der Körperhöhle für den operativen Eingriff bildet. Der Trokardorn weist eine distale Spitze auf, die zur Penetration des Körpergewebes, z.B. der Baudecke, dient, um die Öffnung zu schaffen. Die Spitze des Trokardorns kann dabei scharf, schneidend oder stumpf ausgebildet sein. Eine scharfe Spitze hat beispielsweise die Form einer dreikantigen Pyramide. Schneidende Spitzen weisen eine Klinge auf, die eine Gewebeinzision schafft, welche anschließend durch die konische Spitze dilatiert wird. Stumpfe Spitzen sind distal abgerundet, so dass sehr hohe Penetrationsdrucke für die Eröffnung der Gewebeschichten notwendig sind. Danach bewirken diese im Wesentlichen nur eine Dilatation einer zuvor erzeugten Läsion.

Bei diesen Trokaren, insbesondere den spitzen und schneidenden Trokaren besteht die Gefahr, dass bei der Penetration der Bauchdecke durch Verwachsungen am Peritoneum anhaftende innere Organe, z.B. Darm bzw. Blutgefäße in der Bauchdecke oder in dem Retroperitoneum verletzt werden. Um dieses Risiko zu reduzieren werden sogenannte optische Trokare verwendet, wie sie beispielsweise in der US 5,685,820 A beschrieben sind. Bei diesen optischen Trokaren ist die distale Spitze als hohler durchsichtiger Kegel ausgebildet, der von innen durch eine Optik beobachtbar ist, welche in einem koaxial in dem Trokardorn verlaufenden Optikkanal aufgenommen ist. Der optische Trokar ermöglicht durch die durchsichtige Spitze eine dreidimensionale Sicht auf die Gewebeschichten der Bauchdecke, durch welche die Spitze des Trokars hindurchtritt. Dadurch bekommt der Operateur ein Gefühl für die Bewegung, die Geschwindigkeit und die Position der Trokarspitze bei der Penetration. Insbesondere kann vor der Penetration des Peritoneums erkannt werden, ob Adhäsionen zwischen Darm und Peritoneum an der Insertionsstelle vorhanden sind. Problematisch bleiben die hohen Penetrationsdrücke bei der Penetration der Fascie und des Peritoneums. Diese Penetrationsdrücke könnten zwar durch schneidende Klingen an der Trokarspitze reduziert werden, dies würde jedoch wiederum die Gefahr einer Verletzung des Darmes bei der Penetration erhöhen. Zur Reduktion des Penetrationsdruckes wird der bekannte optische Trokar mit oder ohne schabende Kufen als Kompromiss zwischen Druckminderung und Verletzungsrisiko verwendet, wobei auch in diesem Falle relativ hohe Penetrationsdrucke und permanent rotierende Bewegungen des Trokars erforderlich sind. Auch hierbei entsteht der sogenannte Tentingeffekt, bei welchem die Gewebeschichten, die einen hohen Penetrationsdruck erfordern, von der Trokarspitze zeltartig in das Abdomen gedrückt werden und sich dicht dem Retroperitoneum nähern können. Beim Eröffnen dieser Schichten geben diese dann plötzlich dem Penetrationsdruck nach und die Spitze dringt schlagartig in das Abdomen ein und der Operateur kann Schwierigkeiten haben, die Bewegung des Trokars zu kontrollieren, um nicht mit der Spitze des Trokars innere Organe oder große Gefäße des Retroperitoneums zu verletzen. Um dieses Problem zu reduzieren, wird von zahlreichen Operateuren die Minilaparotomie eingesetzt. Dabei wird über einen Hautschnitt die Bauchdecke in klassischer Weise mit dem Skalpell aufpräpariert und dann eine Trokarhülse in das eröffnete Peritoneum eingesetzt.

Problematisch ist die Abdichtung des Pneumoperitoneums, da die entstandene Öffnung in der Bauchdecke größer ist als dies bei einem Trokareinstich der Fall wäre. Die offene Inzision der Bauchdecke widerspricht dabei allerdings der Zielsetzung der minimal-invasiven Operationstechnik.

Aus der US 2008/0294184 A1 ist ein Trokarsystem bekannt, welches aus einem Trokardorn und einer Trokarhülse besteht. Der Trokardorn ist mit einem koaxial verlaufenden Optikkanal ausgebildet, in welchen eine Optik einführbar ist. Die distale Spitze des Trokardorns ist durchsichtig ausgebildet, so dass sie von innen mittels der eingeführten Optik beobachtet werden kann. Dadurch ist es möglich, dass an der Trokarspitze und unmittelbar vor der Trokarspitze anliegende Gewebe unter Sicht zu penetrieren. Um die Penetrationsöffnung zu erweitern und das Eindringen der Trokarspitze zu erleichtern, sind im distalen Ende der Trokarhülse Klingen schwenkbar gelagert, die seitlich aus der Umfangskontur der Trokarhülse ausgeschwenkt werden können, um das an dem distalen Ende der Trokarhülse anliegende Gewebe zu durchtrennen. Das Verschwenken der Klinge wird mittels eines stabförmigen Betätigungselements bewirkt, welches axial verschiebbar in einem Arbeitskanal gelagert ist, der durchgehend von proximal nach distal in der Wandung der Trokarhülse führt. Mittels der Klingen kann die mittels der distalen Spitze des Trokardorn erzeugte Perforationsöffnung erweitert werden, um das Eindringen der Trokarhülse mit dem größeren Außendurchmesser zu erleichtern. Die erste Penetration des Gewebes muss dabei durch die Spitze des Trokardorns bewirkt werden. Aus der WO 2006/050047 A2 ist ein Instrument für die Hirnchirurgie bekannt mit einem hohlen Obturator, dessen stumpfes distales Ende durchsichtig ist und mittels einer in den Obturator einführbaren Optik von innen beobachtet werden kann. In der Wandung des Obturators ist ein axial durchgehender Kanal ausgebildet, durch welchen eine Sonde einführbar ist, die distal aus dem Obturator austreten kann, um die Position der Obturatorspitze zu ertasten. Der stumpfe Obturator ermöglicht es, die weiche Hirnmasse auseinander zu drücken. Es handelt sich nicht um ein Trokarsystem mit einer distalen penetrierenden Spitze des Trokardorns. Der die Sonde führende Kanal ist in der Wandung des Obturators ausgebildet und nicht in der dem Obturator umschließenden Hülse.

Der Erfindung liegt die Aufgabe zu Grunde, ein Trokarsystem zur Verfügung zu stellen, welches die Vorteile des optischen Trokars ausnützt, ohne hohe Drücke bei der Gewebepenetration zu benötigen.
Diese Aufgabe wird erfindungsgemäß gelöst durch ein Trokarsystem mit den Merkmalen des Patentanspruchs 1.
Vorteilhafte Ausführungen der Erfindung sind in den Unteransprüchen angegeben.
Der wesentliche Gedanke der Erfindung besteht darin, den Trokar nicht nur als passives Werkzeug zu verwenden, welches manuell durch eine axiale Kraft und gegebenenfalls durch eine Rotationsbewegung durch die Gewebeschichten geführt wird. Dem Trokar können vielmehr erfindungsgemäß verschiedene aktive chirurgische Instrumente zugeordnet werden, die durch wenigstens einen Arbeitskanal hindurchgeführt und an der distalen Spitze des Trokars ausgefahren werden können. Dadurch ist es für den Operateur möglich, unmittelbar an der distalen Trokarspitze aktiv chirurgisch tätig zu werden, ohne dass zusätzlich zu dem Trokar ein weiterer Zugang geschaffen werden muss. Über die Optik und die durchsichtige distale Spitze des Trokardorns kann der Operateur die chirurgischen Vorgänge mittels der durch die Arbeitskanäle ausgefahrenen Instrumente unter Sicht durchführen. Es steht eine große Anzahl unterschiedlicher Instrumente in einer miniaturisierten Ausführung zur Ver-fügung, die sich für das Hindurchführen durch die Arbeitskanäle eignen. Eine entsprechende Vielzahl von unterschiedlichen Eingriffen wird durch das erfindungsgemäße Trokarsystem ermöglicht.

Für das Einführen des Trokars durch die verschiedenen Gewebeschichten, insbesondere der Bauchdecke, kann eine miniaturisierte Schere oder ein Messer an der distalen Trokarspitze ausgefahren werden, um die jeweilige Gewebeschicht vor der distalen Spitze zu durchtrennen oder einzuschneiden. Insbesondere die widerstandsfähigen Gewebeschichten, wie die Fascie und das Peritonemum können auf diese Weise unter Sicht durch eine kleine Inzision geöffnet werden, sodass anschließend die Spitze des Trokardorns in diese Inzision eindringen kann und ohne stärkeren Druck und insbesondere ohne Tentingeffekt die Inzision dilatieren und die Gewebeschicht penetrieren kann. Ähnlich der Technik der offenen Laparotomie können somit die jeweils an der distalen Spitze des Trokars anliegenden Gewebeschichten visualisiert und präpariert werden, um ein nahezu druckloses und damit risikoloses Penetrieren der Gewebeschichten zu ermöglichen. Die Semitransparenz des Peritoneums erlaubt dabei das Erkennen von Adhäsionen mittels des optischen Trokars vor der Eröffnung. Dies ist bei der offenen Minilaparotomie nicht möglich.

Weiter können durch die Arbeitskanäle Pinzetten oder Fasszangen hindurchgeführt werden, um das Gewebe an der distalen Trokarspitze festzuhalten, was insbesondere für eine Inzision mittels einer durch einen weiteren Arbeitskanal eingeführten Schere oder Klinge vorteilhaft sein kann.

Bei dem ersten Einstich ist es weiter in vorteilhafter Weise möglich, durch einen Arbeitskanal eine miniaturisierte Veressnadel einzuführen. Mit dieser Veressnadel kann unter Sicht das Peritonemum durchstoßen werden, um dann über die Veressnadel den Bauchraum zu insufflieren.

Weiter können durch die Arbeitskanäle Klemmen oder Koagulationsinstrumente hindurchgeführt und an der distalen Spitze ausgefahren werden. Mit diesen Instrumenten können Gefäße abgeklemmt bzw. koaguliert werden.

Weiter können auch miniaturisierte Morcellatoren über die distale Spitze ausgefahren werden.

Weiter ist es auch möglich, einen miniaturisierte Digitalkamera durch einen Arbeitskanal einzuführen, um z. B. den Operationsvorgang zu dokumentieren. Es können auch Lichtleiter, Beleuchtungssysteme oder Optiken durch einen Arbeitskanal geführt werden.

Das erfindungsgemäße Trokarsystem ermöglicht somit die Penetration insbesondere der Bauchdecke, wozu beispielsweise der Trokar eingestochen wird, bis seine distale Spitze die Fascie erreicht. Über einen Arbeitskanal wird eine Klemme ausgefahren, die die Fascie hält, während durch einen anderen Arbeitskanal eine Schere ausgefahren wird, die die Fascie eröffnet. Dies geschieht unter Sicht durch die durchsichtige Spitze des Trokardorns. Die Spitze des Trokardorns wird nun in die erzeugte Öffnung der Fascie eingebracht, wobei die weitere Dilatation atraumatisch mit geringem Penetrationsdruck erfolgt. Erreicht die Trokarspitze das Peritoneum und werden keine Adhäsionen festgestellt, so kann in entsprechender Weise das Peritoneum mittels Schere und gegebenenfalls Klemme eröffnet werden. Auch hierbei ist kein nennenswerter Penetrationdruck erforderlich, sodass der Tentingeffekt mit seinen Risiken vermieden wird. Dieses Vorgehen ist analog zu den gewohnten präparativen Schritten bei der offenen Minilaparotomie. Zum Unterschied zu diesem bekannten präparativen Vorgehen ist jedoch kein größerer Schnitt erforderlich, als für das Einbringen des Trokars notwendig, um die Gewebeschichten unter Sicht präparieren zu können. Alternativ kann bei Erreichen des Peritoneums auch eine Veressnadel durch einen Arbeitskanal ausgefahren werden, mit welcher dann das Peritoneum unter Sicht penetriert wird, um dann CO₂ in das Abdomen zu insufflieren. Sobald durch die Insufflation das Peritoneum von den Därmen distanziert ist, wird die Veressnadel zurückgezogen und die Spitze des Trokars wird in die durch die Veressnadel gebildete Öffnung eingebracht, um diese unter geringem Druck atraumatisch zu dilatieren.

Mit dem erfindungsgemäßen Trokarsystem wird in die erzeugte Körperöffnung die Trokarhülse eingesetzt, die dann nach Entfernen des Trokardorns als Zugangskanal für den nachfolgenden minimal-invasiven Eingriff dient. Ebenso ist es möglich, eine Single-Port-Operation mittels des Trokarsystems durchzuführen. In diesem Falle verbleibt der Trokardorn mit der Optik in der Trokarhülse und die Arbeitskanäle dienen als einziger Zugang für den anschließenden minimal-invasiven Eingriff, wozu die Instrumente für den Eingriff durch die Arbeitskanäle eingeführt werden. Der Eingriff kann dabei unter Sicht durch die durchsichtige Spitze des Trokardorns erfolgen. Alternativ kann nach dem Einsetzen der Trokarhülse der Trokardorn gegen eine Optik getauscht werden, um nach der Insufflation freie Sicht auf das Operationsfeld zu erhalten.

Die mit dem erfindungsgemäßen Trokarsystem verwendbaren Instrumente sind im Wesentlichen an sich bekannte chirurgische Miniaturinstrumente. Diese weisen ein an der distalen Spitze des Trokars ausfahrbares Arbeitselement auf, während an dem proximal außerhalb des Arbeitskanals verbleibenden Ende die proximalen Betätigungselemente des Miniaturinstrüments angeordnet sind. Die Instrumente können mit einem starren oder einem flexiblen oder semiflexiblen Schaft ausgebildet sein. Bei semiflexiblen Instrumenten ist es möglich, diese so elastisch vorzuspannen, dass sich ihre distalen Arbeitselemente beim Austritt aus der distalen Spitze des Trokars gegen die Mittelachse des Trokars biegen, um ein präparatives Arbeiten unmittelbar vor der durchsichtigen Spitze zu ermöglichen.

Alternativ kann in dem Arbeitskanal auch ein Führungsröhrchen axial verschiebbar und drehbar angeordnet sein, durch welches das Miniaturinstrument durchgeführt wird. Das Führungsröhrchen ist in seinem distalen Endbereich elastisch auf Biegung vorgespannt. Vorzugsweise besteht das Führungsröhrchen aus einer Memory-Legierung mit superelastischen Eigenschaften, z. B. aus Nitinol. Wird das Führungsröhrchen distal aus dem Arbeitskanal herausgeschoben, so krümmt sich sein distales Ende aus seiner durch den Arbeitskanal festgelegten Längsachse, wobei der Ablenkwinkel gegenüber der Längsachse zunimmt, je weiter das distale Ende des Führungsröhrchens aus dem Arbeitskanal austritt. Durch Drehen des Führungsröhrchens in dem Arbeitskanal kann dabei die Richtung der Ablenkung um die Längsachse gedreht werden. Durch axiales Verschieben und Drehen des Führungsröhrchens kann somit die Austrittsrichtung und Positionierung des distalen Arbeitselements des Miniaturinstruments dreidimensional gesteuert werden. Am proximalen Ende vorgesehene Stellmittel erlauben die axiale und rotatorische Bewegung des Führungsröhrchens in dem Arbeitskanal.

Um ein Entweichen des Gases aus dem insufflierten Bauchraum zu verhindern, können die Arbeitskanäle proximal durch ein Ventil verschließbar sein, wenn sich kein Instrument in dem Arbeitskanal befindet. Ein solches Ventil kann insbesondere durch eine an sich bekannte Lippendichtung gebildet sein, die ein Durchtreten eines Instruments erlaubt und ein eingesetztes Instrument an dessen Außenumfang abdichtet.

Weiter können unbenutzte Arbeitskanäle durch einen Mandrin verschlossen werden, der die distale Austrittsöffnung des Arbeitskanals verschließt, sodass insbesondere eine Verunreinigung des Arbeitskanals vermieden wird.

Die in dem Trokarsystem von proximal nach distal durchgehenden Arbeitskanäle können in unterschiedlicher Weise hergestellt werden. Gemeinsam ist den verschiedenen Ausführungen, dass die Innenwandung der Arbeitskanäle zumindest in einem Teilbereich ihres Querschnitts durch die Trokarhülse gebildet ist.

In einer Ausführung ist der wenigstens eine Arbeitskanal als axial durchgehende Bohrung in der Wandung der Trokarhülse ausgebildet. Das Material der Trokarhülse umschließt somit den Arbeitskanal an dessen gesamtem Umfang. Diese Ausführung ist herstellungstechnisch aufwendiger, hat jedoch den Vorteil, dass die an ihrem gesamten Umfang geschlossenen Arbeitskanäle in der Trokarhülse auch dann noch zur Verfügung stehen, wenn der Trokardorn nach dem Setzen der Trokarhülse herausgezogen wird. Die Arbeitskanäle der Trokarhülse können dann zusätzlich zu dem Innenlumen Trokarhülse für den operativen Eingriff genutzt werden. Dies kann insbesondere bei der Single-Port-Operationstechnik von Vorteil sein.

Herstellungstechnisch einfacher ist es, wenn der wenigstens eine Arbeitskanal zwischen der Außenmantelfläche des Trokardorns und der Innenwandfläche der Trokarhülse gebildet ist.

Dabei kann der wenigstens eine Arbeitskanal als eine axial durchgehende Nut in der Innenwandfläche der Trokarhülse ausgebildet sein, die durch die Außenmantelfläche des Trokardorns abgeschlossen ist. Die axial durchgehende Nut kann einfach hergestellt werden. Die Wandstärke der Trokarhülse kann geringer sein als bei der ersten Ausführung. Vorteilhaft ist weiter, dass ein beliebiger Trokardorn verwendet werden kann.

Alternativ kann der wenigstens eine Arbeitskanal durch eine axial durchgehende Nut in der Außenmantelfläche des Trokardorns gebildet sein, die dann durch die Innenwandfläche der Trokarhülse verschlossen wird. Bei dieser Ausführung ist der Trokardorn erfindungsgemäß ausgebildet, während eine herkömmliche Trokarhülse verwendet werden kann. Weiter ist von Vorteil, dass hierbei die geringste Wandstärke der Trokarhülse erforderlich ist.

In einer anderen Ausführung kann der wenigstens eine Arbeitskanal durch axial durchgehende Nuten sowohl in der Außenmantelfläche des Trokardorns als auch in der Innenwandfläche der Trokarhülse gebildet werden. Die Nuten kommen dabei in ihrer Umfangswinkelposition zur Deckung, sodass die Nut in dem Trokardorn und die Nut in der Trokarhülse jeweils den halben Innenquerschnitt des Arbeitskanals bilden.

Weiter ist es möglich, den wenigstens einen Arbeitskanal durch ein außen auf die Mantelfläche der Trokarhülse angebrachtes Röhrchen zu bilden.

Schließlich ist auch eine Ausführung möglich, bei welcher zwischen der Außenmantelfläche des Trokardorns und der Innenwandfläche der Trokarhülse ein kreisringförmiger Spalt bleibt, in welchen wenigstens eine Schale eingesetzt wird, die in einem Umfangswinkelbereich axial durchgehend den Spalt frei lässt, wodurch der Arbeitskanal gebildet ist.

Die Arbeitskanäle verlaufen im allgemeinen achsparallel zu der Trokarhülse oder in einem Winkel zur Instrumentenachse oder auch schraubenförmig um die Instrumentenachse. Im proximalen Eintrittsbereich und/oder im distalen Austrittsbereich können die Arbeitskanäle jedoch auch von der achsparallelen Richtung nach außen abgewinkelt verlaufen.

Vorzugsweise kann in der Wandung der Trokarhülse eine axial durchgehende Bohrung als Insufflationskanal angeordnet sein. Dadurch ist eine einfache Möglichkeit gegeben, eine Insufflation während des operativen Eingriffs durchzuführen, was insbesondere bei der Single-Port-Operationstechnik von Vorteil ist. Selbstverständlich kann die Insufflation auch durch das Innenlumen der Trokarhülse erfolgen, wie dies bei bekannten Trokarsystemen üblich ist.

An der Trokarhülse kann in der Regel in an sich bekannter Weise ein Ventilgehäuse angebracht sein, welches die Trokarhülse proximal verschließt, wenn kein Trokardorn oder Instrument in die Trokarhülse eingeführt ist. Wird der Trokardorn oder ein Operationsinstrument durch die Trokarhülse eingeführt, so werden diese an ihrem Umfang abgedichtet, um ein Entweichen des Insufflationsgases zu verhindern. Die proximalen Eintrittsenden der Arbeitskanäle befinden sind außerhalb des Ventilgehäuses, sodass die jeweiligen Instrumente unabhängig von dem Ventilgehäuse durch die Arbeitskanäle hindurchgeführt werden können.

Im Folgenden wird die Erfindung anhand von in der Zeichnung dargestellten Ausführungsbeispielen näher erläutert. Es zeigen:
- Fig. 1: einen Axialschnitt durch das Trokarsystem in einer ersten Ausführung,
- Fig. 2: einen Querschnitt des Trokarsystems gemäß der Schnittlinie A-A in Figur 1,
- Fig. 3: einen entsprechenden Querschnitt des Trokarsystems in einer zweiten Ausführung,
- Fig. 4: einen entsprechenden Querschnitt des Trokarsystems in einer dritten Ausführung,
- Fig. 5: einen entsprechenden Querschnitt des Trokarsystems in einer vierten Ausführung,
- Fig. 6: einen entsprechenden Querschnitt des Trokarsystems in einer fünften Ausführung und
- Fig. 7: einen entsprechenden Querschnitt des Trokarsystems in einer sechsten Ausführung.

In der Zeichnung und in der nachfolgenden Beschreibung sind nur die Teile des Trokarsystems dargestellt, die erfindungsgemäß ausgebildet sind. Im Übringen entspricht das Trokarsystem dem bekannten Stand der Technik, wobei alle aus dem Stand der Technik bekannten Abwandlungen miterfasst sind.

Das Trokarsystem weist einen Trokardorn 10 auf. Der Trokardorn 10 hat die Form eines starren langgestreckten zylindrischen Rohres, welches aus Metall oder Kunststoff gefertigt ist. Das Innenlumen des Trokardorns 10 bildet einen koaxial vom proximalen Ende zum distalen Ende führenden Optikkanal 12. Die distale Spitze 14 des Trokardorns 10 hat z.B. die Form eines Kegels mit abgerundet stumpfer Spitze. Der Kegelmantel kann gegebenenfalls auch ballig ausgewölbt sein. Die kegelförmige Spitze 14 ist im Inneren hohl und besteht aus einem dünnwandigen durchsichtigen Material, insbesondere aus einem durchsichtigen Kunststoff. In den Optikkanal 12 des Trokardorns 10 ist eine Optik einsetzbar, die beispielsweise als Stablinsenoptik oder mit einem Kamerachip ausgebildet sein kann und ein Beleuchtungssystem integriert haben kann. Bei eingesetzter Optik befindet sich deren distales Ende etwa im Bereich der Basisfläche der kegelförmigen Spitze 14. Durch die Optik kann die distale Spitze 14 von innen ausgeleuchtet und beobachtet werden. Dadurch ist es möglich, das an der Außenseite der distalen Spitze 14 anliegende Gewebe zu beobachten.

Auf den Trokardorn 10 kann eine Trokarhülse 16 aufgeschoben werden. An dem proximalen Ende der Trokarhülse 16 ist in an sich bekannter und deshalb nicht näher erläuterten Weise ein Ventil angeordnet, welches die Trokarhülse 16 abdichtend verschließt, wenn sich in der Trokarhülse 16 kein Instrument befindet. Wird der Trokardorn 10 oder ein sonstiges Instrument durch die Trokarhülse 16 eingeführt, so werden diese an ihrem Umfang durch das Ventil abgedichtet.

In der Trokarhülse 16 kann ein Insufflationskanal 18 ausgebildet sein. Der Insufflationskanal 18 verläuft als achsparallele Bohrung in der Wandung der Trokarhülse 16. An dem proximalen Ende des Insufflationskanals 18 befindet sich ein Anschluss 20 für das Insufflationsgas. Distal mündet der Insufflationskanal 18 offen an dem distalen Ende der Trokarhülse 16. Alternativ kann der Anschluss 20 auch in das Innenlumen der Trokarhülse 16 führen, sodass das Innenlumen den Insufflationskanal bildet.

Ist die Trokarhülse 16 auf den Trokardorn 10 aufgeschoben, so schließt das distale Ende der Trokarhülse 16 am proximalen Ende der Spitze 14 des Trokardorns 10 an, sodass die Außenkontur der Spitze 14 stufenlos in den Außenumfang der Trokarhülse 16 übergeht.

Das Trokarsystem weist wenigstens einen Arbeitskanal 22 auf, der in der Trokarhülse 16 von proximal nach distal verläuft. Der Arbeitskanal 22 verläuft im allgemeinen achsparallel zur Mittelachse der Trokarhülse 16. Am proximalen Ende weist der Arbeitskanal 22 eine Eintrittsöffnung 24 auf, die sich außerhalb des Ventils befindet, sodass sie frei zugänglich ist. Die Eintrittsöffnung 24 kann gegen die achsparallele Richtung abgewinkelt sein. Am distalen Ende mündet der Arbeitskanal 22 in eine freie Austrittsöffnung 26 im Bereich des distalen Endes der Trokarhülse 16. Auch die Achsrichtung der Austrittsöffnung 26 kann gegen die achsparallele Richtung abgewinkelt sein. In den dargestellten Ausführungsbeispielen sind jeweils zwei Arbeitskanäle 22 diametral zueinander angeordnet. Es ist jedoch auch möglich, dass nur ein Arbeitskanal 22 oder auch mehr als zwei Arbeitskanäle 22 vorgesehen sind. Sind zwei oder mehr Arbeitskanäle 22 vorgesehen, so sind diese vorzugsweise im gleichem gegenseitigen Winkelabstand angeordnet.

Die Arbeitskanäle 22 dienen zum Einführen von an sich bekannten Miniaturinstrumenten. Diese Miniaturinstrumente sind beliebige, dem jeweiligen Anwendungsfall entsprechende Instrumente, wie sie an sich bekannt sind. Die Miniaturinstrumente weisen einen langgestreckten starren,flexiblen oder semiflexiblen Schaft auf, an dessen distalem Ende jeweils ein Arbeitselement angeordnet ist, welches mittels eines am proximalen Ende des Schaftes angeordneten Betätigungselements betätigt werden kann. Das Miniaturinstrument wird von dem proximalen Ende her in den Arbeitskanal 22 eingeführt, wobei es an der proximalen Eintrittsöffnung 24 abgedichtet werden kann. Ein nach außen abgewinkelter Verlauf der Eintrittsöffnung 24 erleichtert das Einführen des Miniaturinstruments seitlich vor dem Ventil der Trokarhülse 16. Das Miniaturinstrument wird in dem Arbeitskanal 22 soweit vorgeschoben, bis das distale Arbeitselement durch die Austrittsöffnung 26 ausgefahren ist und vor der distalen Spitze 14 zum Einsatz kommen kann. Sind die Trokarhülse 16 und die Arbeitskanäle 22 aus einem durchsichtigen Kunststoff gefertigt, so kann das Einführen und Vorschieben des Miniaturinstruments von außen beobachtet werden.

Bei Bedarf kann in einen nicht genutzten Arbeitskanal 22 ein in der Zeichnung nicht dargestellter Mandrin eingesetzt werden, der mit seinem distalen Ende die Austrittsöffnung 26 flächenbündig verschließt, sodass ein Eindringen von Verunreinigungen in den nicht genutzten Arbeitskanal 22 verhindert wird.

Der wenigstens eine Arbeitskanal 22 kann in unterschiedlicher Weise erzeugt werden.

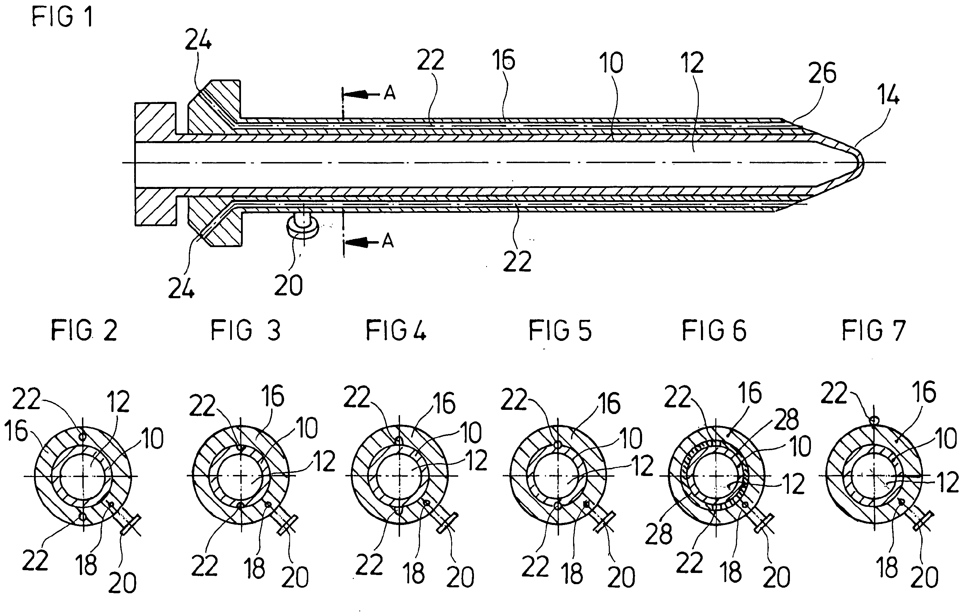

In einer in den Figuren 1 und 2 dargestellten ersten Ausführung ist der wenigstens eine Arbeitskanal 22 als axial durchgehende Bohrung in der massiven Wandung der Trokarhülse 16 ausgebildet. Das Material der Wandung der Trokarhülse 16 umschließt somit den gesamten Querschnittsumfang des Arbeitskanals 22. Diese Ausführung ist besonders geeignet, wenn der Trokardorn 10 nach dem Einsetzen der Trokarhülse 16 durch eine Optik ersetzt wird.

In einem in Figur 3 dargestellten zweiten Ausführungsbeispiel wird der wenigstens eine Arbeitskanal 22 durch eine Nut gebildet, die axial in der Außenmantelfläche des Trokardorns 10 verläuft. Der an der Außenseite des Trokardorns 10 offene Umfangsbereich des Querschnitts der Nut wird durch die Innenwandfläche der aufgeschobenen Trokarhülse 16 abgeschlossen, um den am Umfang geschlossenen Arbeitskanal 22 zu bilden.

In einer in Figur 4 gezeigten dritten Ausführung wird der wenigstens eine Arbeitskanal 22 durch eine axial durchgehende Nut gebildet, die in der Innenwandfläche der Trokarhülse 16 ausgebildet ist. Der an der Innenseite der Trokarhülse 16 offene Bereich der Nut wird durch die Außenmantelfläche des Trokardorns 10 geschlossen, um den an seinem gesamten Querschnittsumfang geschlossenen Arbeitskanal 22 zu bilden.

In einer in Figur 5 gezeigten vierten Ausführung sind sowohl an der Außenmantelfläche des Trokardorns 10 als auch an der Innenwandfläche der Trokarhülse 16 jeweils axial verlaufende Nuten ausgebildet, die in ihrer Winkelposition bezüglich der Trokarachse zur Deckung kommen. Die beiden zur Deckung kommenden Nuten ergänzen sich zu dem Querschnitt des wenigstens einen Arbeitskanals 22.

In einer in Figur 6 gezeigten fünften Ausführung ist der Außendurchmesser des Trokardorns 10 etwas kleiner als der Innendurchmesser der Trokarhülse 16, sodass zwischen der Innenwandfläche der Trokarhülse 16 und der Außenmantelfläche des Trokardorns 10 ein kreisringförmiger Spalt frei bleibt. In diesen Spalt sind eine der Anzahl der Arbeitskanäle 22 entsprechende Anzahl von kreiszylindrischen Schalen 28 eingesetzt. In Umfangsrichtung bleibt zwischen den Schalen 28 ein Umfangswinkelbereich frei, wodurch die axial durchgehenden Arbeitskanäle 22 gebildet werden. Dabei wird der Arbeitskanal 22 an seiner radialen Außenseite durch die Innenwandfläche der Trokarhülse 16, an seiner radialen Innenseite durch die Außenumfangsfläche des Trokardorns 10 und in seinen beiden Umfangsseitenflächen durch die Schalen 28 gebildet.

In einer in Figur 7 gezeigten sechsten Ausführung ist der wenigstens eine Arbeitskanal 22 durch ein Röhrchen gebildet, welches außen an der Außenmantelfläche der Trokarhülse 16 angebracht ist, z. B. angeklebt, angepunktet oder in die Mantelfläche eingegossen ist.

### Bezugszeichenliste

- 10: Trokardorn
- 12: Optikkanal
- 14: Spitze
- 16: Trokarhülse
- 18: Insufflationskanal
- 20: Anschluss
- 22: Arbeitskanal
- 24: Eintrittsöffnung
- 26: Austrittsöffnung
- 28: Schalen

## Patentansprüche

1. Trokarsystem mit einem Trokardorn (10), mit einer Trokarhülse (16), mit einem koaxial in dem Trokardorn (10) verlaufenden Optikkanal (12) zur Aufnahme einer Optik, mit einer hohlen durchsichtigen distalen Spitze (14) des Trokardorns (10), die mittels der Optik von innen beobachtbar ist und mit wenigstens einem Arbeitskanal (22), der durchgehend von proximal nach distal in dem Trokarsystem führt und im Bereich des distalen Endes in einer Austrittsöffnung (26) mündet, wobei die Innenwandung des wenigstens einen Arbeitskanals (22) zumindest in einem axialen Teilbereich ihres Umfangs durch die Trokarhülse (16) gebildet ist,
**dadurch gekennzeichnet, dass** wenigstens ein Miniaturinstrument durch den wenigstens einen Arbeitskanal (22) in der Weise einführbar ist, dass ein distales Arbeitselement des Miniaturinstruments distal aus der Austrittsöffnung (26) des Arbeitskanals (22) austritt und vor der distalen Spitze (14) zum Einsatz kommen kann, während ein proximales Betätigungselement des Miniaturinstruments sich proximal außerhalb der Eintrittsöffnung (24) des Arbeitskanals (22) befindet.

2. Trokarsystem nach Anspruch 1,
**dadurch gekennzeichnet, dass** der wenigstens eine Arbeitskanal (22) als durchgehende Bohrung in der Wandung der Trokarhülse (16) ausgebildet ist, wobei die Trokarhülse (16) die Innenwandung des Arbeitskanals (22) in dessen gesamten Umfang bildet.

3. Trokarsystem nach Anspruch 1,
**dadurch gekennzeichnet, dass** der wenigstens eine Arbeitskanal (22) als axial verlaufende Nut in der Innenwandfläche der Trokarhülse (16) ausgebildet ist, wobei der offene Umfangsbereich der Nut durch die Außenmantelfläche des Trokardorns (10) abgeschlossen ist.

4. Trokarsystem nach Anspruch 1,
**dadurch gekennzeichnet, dass** der wenigstens eine Arbeitskanal (22) als axial verlaufende Nut in der Außenmantelfläche des Trokardorns (10) ausgebildet ist, wobei der offene Umfangsbereich der Nut durch die Innenwandfläche der Trokarhülse (16) abgeschlossen ist.

5. Trokarsystem nach Anspruch 1,
**dadurch gekennzeichnet, dass** der wenigstens eine Arbeitskanal (22) durch axial verlaufende Nuten in der Außenmantelfläche des Trokardorns (10) und in der Innenwandfläche der Trokarhülse (16) zusammengesetzt ist, die in ihrer Winkelposition zur Deckung kommen.

6. Trokarsystem nach Anspruch 1,
**dadurch gekennzeichnet, dass** zwischen die Außenmantelfläche des Trokardorns (10) und die Innenwandfläche der Trokarhülse (16) wenigstens eine kreiszylindrische Schale (28) eingesetzt ist, die wenigstens einen Umfangswinkelbereich axial durchgehend frei lässt, wobei der wenigstens eine Umfangswinkelbereich innen durch die Außenmantelfläche des Trokardorns (10) und außen durch die Innenwandfläche der Trokarhülse (16) abgeschlossen ist und den wenigstens einen Arbeitskanal (22) bildet.

7. Trokarsystem nach Anspruch 1,
**dadurch gekennzeichnet, dass** der wenigstens eine Arbeitskanal 822) durch ein an der Außenmantelfläche der Trokarhülse (16) abgebrachtes Röhrchen gebildet ist.

8. Trokarsystem nach einem der vorhergehenden Ansprüche,
**dadurch gekennzeichnet, dass** in der Wandung der Trokarhülse (16) eine axial durchgehende Bohrung als Insufflationskanal (18) zum Einleiten eines Gases für die Insufflation ausgebildet ist.

9. Trokarsystem nach einem der vorhergehenden Ansprüche,
**dadurch gekennzeichnet, dass** an der Trokarhülse (16) proximal ein Ventil angeordnet ist, welches ein abgedichtetes Durchtreten des Trokardorns (10) ermöglicht und die Trokarhülse (16) verschließt, wenn der Trokardorn (10) nicht eingesetzt ist, und dass die proximale Eintrittsöffnung (24) des wenigstens einen Arbeitskanals (22) außerhalb des Ventils liegt.

10. Trokarsystem nach Anspruch 1,
**dadurch gekennzeichnet, dass** das Miniaturinstrument eine Schere, ein Messer, eine Pinzette, eine Klemme, ein Koagulationsinstrument, eine Veressnadel, eine Digitalkamera, ein Lichtleiter, ein Beleuchtungssystem oder eine Optik ist.

11. Trokarsystem nach Anspruch 1,
**dadurch gekennzeichnet, dass** in dem wenigstens einen Arbeitskanal (22) ein Führungsröhrchen axial verschiebbar und drehbar angeordnet ist, durch welches das Miniaturinstrument hindurchführbar ist, und dass das Führungsröhrchen zumindest in seinem distalen Endbereich auf Krümmung elastisch vorgespannt ist.

12. Trokarsystem nach Anspruch 11,
**dadurch gekennzeichnet, dass** das Führungsröhrchen aus einem Memory-Werkstoff mit superelastischen Eigenschaften, z. B. Nitinol besteht.

## Claims

1. Trocar system having a trocar (10), having a trocar sleeve (16), having an optical channel (12), which runs coaxially in the trocar (10) and is intended for accommodating an optical unit, having a hollow transparent distal tip (14) of the trocar (10), it being possible for the distal tip to be observed from within by means of the optical unit, and having at least one working channel (22), which leads continuously from the proximal end to the distal end in the trocar system and opens out in an exit opening (26) in the region of the distal end, wherein the inner wall of the at least one working channel (22) is formed by the trocar sleeve (16) at least in an axial sub-region of its circumference,
**characterized in that** at least one miniature instrument can be introduced through the at least one working channel (22) such that a distal working element of the miniature instrument exits distally from the exit opening (26) of the working channel (22) and can be used upstream of the distal tip (14), while a proximal actuating element of the miniature instrument is located proximally outside the entry opening (24) of the working channel (22).

2. Trocar system according to claim 1,
**characterized in that** the at least one working channel (22) is designed in the form of a continuous bore in the wall of the trocar sleeve (16), wherein the trocar sleeve (16) forms the inner wall of the working channel (22) over the entire circumference of the latter.

3. Trocar system according to claim 1,
**characterized in that** the at least one working channel (22) is designed in the form of an axially running groove in the inner wall surface of the trocar sleeve (16), wherein the open circumferential region of the groove is closed off by the outer lateral surface of the trocar (10) .

4. Trocar system according to claim 1,
**characterized in that** the at least one working channel (22) is designed in the form of an axially running groove in the outer lateral surface of the trocar (10), wherein the open circumferential region of the groove is closed off by the inner wall surface of the trocar sleeve (16).

5. Trocar system according to claim 1,
**characterized in that** the at least one working channel (22) is formed by axially running grooves in the outer lateral surface of the trocar (10) and in the inner wall surface of the trocar sleeve (16), said grooves, in their angled position, coinciding.

6. Trocar system according to claim 1,
**characterized in that** the outer lateral surface of the trocar (10) and the inner wall surface of the trocar sleeve (16) have inserted between them at least one circular-cylindrical shell (28), which leaves at least one circumferential-angle region continuously free in the axial direction, wherein the at least one circumferential-angle region is closed off on the inside by the outer lateral surface of the trocar (10) and on the outside by the inner wall surface of the trocar sleeve (16) and forms the at least one working channel (22).

7. Trocar system according to claim 1,
**characterized in that** the at least one working channel 822) is formed by a tube fitted on the outer lateral surface of the trocar sleeve (16).

8. Trocar system according to one of the preceding claims,
**characterized in that** the wall of the trocar sleeve (16) contains an axially continuous bore in the form of an insufflation channel (18) for the introduction of a gas for insufflation purposes.

9. Trocar system according to one of the preceding claims,
**characterized in that** a valve is arranged proximally on the trocar sleeve (16), said valve providing for sealed through-passage of the trocar (10) and closing the trocar sleeve (16) when the trocar (10) is not inserted, and **in that** the proximal entry opening (24) of the at least one working channel (22) is located outside the valve.

10. Trocar system according to claim 1,
**characterized in that** the miniature instrument is a pair of scissors, a blade, a pair of tweezers, a clamp, a coagulation instrument, a Veress needle, a digital camera, a light guide, an illumination system or an optical unit.

11. Trocar system according to claim 1,
**characterized in that** a guide tube is arranged in an axially displaceable and rotatable manner in the at least one working channel (22), it being possible for the miniature instrument to be guided through said guide tube, and **in that** the guide tube is prestressed elastically under curvature at least in its distal end region.

12. Trocar system according to claim 11,
**characterized in that** the guide tube consists of a memory material with superelastic properties, e.g. Nitinol.

## Revendications

1. Système de trocart comprenant un mandrin de trocart (10), un manchon de trocart (16), un canal optique (12) s'étendant coaxialement dans le mandrin de trocart (10) pour recevoir une optique, et une pointe distale (14) transparente creuse du mandrin de trocart (10) qui peut, au moyen de l'optique être observée de l'intérieur, ainsi qu'au moins un canal de travail (22) qui s'étend en continue dans le système de trocart, de l'extrémité distale à l'extrémité proximale et, débouche dans la zone de l'extrémité distale par une ouverture de sortie (26), la paroi interne du canal de travail (22) étant formée au moins dans une zone partielle axiale de sa périphérie par le manchon de trocart (16),
**caractérisé en ce qu'**
au moins un instrument miniaturisé peut être introduit au travers du canal de travail (22) de sorte qu'un élément de travail distal de cet instrument miniaturisé puisse sortir à l'extrémité distale par l'ouverture de sortie (26) de ce canal de travail (22) et puisse être mis en oeuvre à l'avant de la pointe distale (14) tandis qu'un élément d'actionnement proximal de l'instrument miniaturisé est situé à l'extrémité proximale en dehors de l'ouverture d'entrée (24) du canal de travail (22) .

2. Système de trocart conforme à la revendication 1,
**caractérisé en ce que**
le canal de travail (22) est réalisé sous la forme d'un perçage traversant dans la paroi du manchon de trocart (16), le manchon de trocart (16) formant la paroi interne du canal de travail (22) sur la totalité de sa
périphérie.

3. Système de trocart conforme à la revendication 1,
**caractérisé en ce que**
le canal de travail (22) est réalisé sous la forme d'une nervure s'étendant axialement dans la surface de la paroi interne du manchon de trocart (16), la zone périphérique ouverte de la rainure étant fermée par la surface enveloppe externe du mandrin de trocart (10).

4. Système de trocart conforme à la revendication 1,
**caractérisé en ce que**
le canal de travail (22) est réalisé sous la forme d'une rainure s'étendant axialement dans la surface enveloppe externe du mandrin de trocart (10), la zone périphérique ouverte de la rainure étant fermée par la surface de la paroi interne du manchon de trocart (16).

5. Système de trocart conforme à la revendication 1,
**caractérisé en ce que**
le canal de travail (22) est réalisé par l'assemblage de rainures s'étendant axialement dans la surface enveloppe externe du mandrin de trocart (10) et dans la surface de la paroi interne du manchon de trocart (16) dont les positions angulaires se chevauchent.

6. Système de trocart conforme à la revendication 1,
**caractérisé en ce qu'**
entre la surface enveloppe externe du mandrin de trocart (10) et la surface de la paroi interne du manchon de trocart (16) est insérée au moins une coquille (28) en forme de cylindre circulaire qui laisse un passage axial libre dans au moins une zone angulaire périphérique, cette zone angulaire périphérique étant fermée à l'intérieur par la surface enveloppe externe du mandrin de trocart (10) et à l'extérieur par la surface de la paroi interne du manchon de trocart (16), et formant le canal de travail (22).

7. Système de trocart conforme à la revendication 1,
**caractérisé en ce que**
le canal de travail (22) est formé par un petit tube inséré sur la surface enveloppe externe du manchon de trocart (16).

8. Système de trocart conforme à l'une des revendications précédentes,
**caractérisé en ce que**
dans la paroi du manchon de trocart (16) est formé un perçage axial traversant sous la forme d'un canal de soufflage (18) permettant l'introduction d'un gaz d'insufflation.

9. Système de trocart conforme à l'une des revendications précédentes,
**caractérisé en ce qu'**
à l'extrémité proximale du manchon de trocart (16) est montée une soupape qui permet un passage étanche du mandrin de trocart (10) et ferme le manchon de trocart (16) lorsque le mandrin de trocart (10) n'est pas inséré, et, l'ouverture d'entrée proximale (24) du canal de travail (22) est située à l'extérieur de la soupape.

10. Système de trocart conforme à la revendication 1,
**caractérisé en ce que**
l'instrument miniature est constitué par des ciseaux, une lame, une pincette, une pince, un instrument de coagulation, une aiguille de suture, une caméra numérique, un conducteur de lumière, un système d'éclairage ou une optique.

11. Système de trocart conforme à la revendication 1,
**caractérisé en ce que**
dans le canal de travail (22) est monté un petit tube de guidage mobile en translation axiale et en rotation, au travers duquel peut passer l'instrument miniaturisé, et le petit tube de guidage est courbé élastiquement sous précontrainte au moins au niveau de sa zone d'extrémité distale.

12. Système de trocart conforme à la revendication 11,
**caractérisé en ce que**
le petit tube de guidage est réalisé en un matériau à mémoire de forme ayant des caractéristiques super-élastiques, notamment en nitinol.
